# EUROPEAN PATENT APPLICATION

(11) **EP 4 796 526 A1**
(43) Date of publication of application: **26.08.2026**
(21) Application number: 24879477.8
(22) Date of filing: 13.09.2024
(51) Int. Cl.: C07C 211/00

(54) **REACTION COMPOSITION, EPOXY RESIN CURING AGENT, EPOXY RESIN COMPOSITION, AND CURED PRODUCT THEREOF**

(30) Priority: 19.10.2023 JP 2023180379
(71) Applicant: MITSUBISHI GAS CHEMICAL COMPANY, INC., Chiyoda-ku Tokyo 100-8324 (JP)
(72) Inventor: AKAI, Yuka, Hiratsuka-shi, Kanagawa 254-0016 (JP); OHNO, Yuma, Hiratsuka-shi, Kanagawa 254-0016 (JP); KOUNO, Kazuki, Hiratsuka-shi, Kanagawa 254-0016 (JP)
(74) Representative: Gille Hrabal Partnerschaftsgesellschaft mbB Patentanwälte
(86) International application number: PCT/JP2024/032989
(87) International publication number: WO 2025/084050

(57) **Abstract**

A reaction composition containing a reaction product of xylylenediamine and a (meth)acrylamide derivative having only one (meth)acrylic group, and an epoxy resin curing agent containing the reaction composition, an epoxy resin composition, and a cured product thereof are provided.

## Description

### Technical Field

The present invention relates to a reaction composition, an epoxy resin curing agent, an epoxy resin composition, and a cured product thereof.

### Background Art

A polyamine compound is known as a kind of epoxy resin curing agent. Epoxy resin compositions using a polyamine compound as an epoxy resin curing agent are used not only in the field of paints such as corrosion-resistant paints for boats and ships, bridges, and onshore and offshore iron structures but also in the field of civil engineering and architecture as lining, reinforcing, and repairing materials for concrete structures, floor materials for architectural structures, linings for water supply and sewerage systems, paving materials, adhesives, and the like.

Among these, for epoxy resin compositions used for paints, it is important to provide coating films having excellent appearance, water resistance, chemical resistance, and physical properties.

Xylylenediamine as a kind of aliphatic polyamine compound can quickly cure an epoxy resin when used as an epoxy resin curing agent and is superior in low-temperature curability, chemical resistance, etc. to other aliphatic polyamines. However, xylylenediamine is likely to absorb carbon dioxide and water vapor in air to generate a carbamic acid salt, and therefore a coating film of an epoxy resin composition using xylylenediamine as an epoxy resin curing agent is likely to blush, which tends to deteriorate the appearance of the coating film.

As methods for improving the blushing of the coating film as described above and other various properties, epoxy resin curing agents prepared by modifying xylylenediamine have been investigated.

For example, Patent Literature 1 discloses that a curing agent composition for epoxy resin containing a polyamine compound, which is a reaction product of a compound having at least one glycidyl group in one molecule and a diamine such as xylylenediamine, a predetermined polyether-modified polysiloxane, and a predetermined amino group-modified polysiloxane can solve a problem that appearance is deteriorated due to blushing caused by a reduction in water resistance, which makes it possible to provide an epoxy resin composition excellent in surface appearance such as transparency, drying efficiency (ease of drying), adhesiveness to base materials, and water resistance.

Patent Literature 2 discloses that an aqueous epoxy resin composition obtained by blending, in a predetermined proportion, an aqueous epoxy resin and a curing agent composition containing a reaction product of epichlorohydrin and xylylenediamine exhibits good workability and provides a coating film having excellent adhesion and chemical resistance.

### Citation List

### Patent Literatures

PTL1: JP 2007-186693 A
PTL2: WO 2020/110601

### Summary of Invention

### Technical Problem

Cured products of epoxy resin compositions generally exhibit poor adhesion to inorganic substances, particularly aluminum. In the conventional art, there has been room for improvement in this regard.

An object of the present invention is to provide a novel reaction composition which, when used as an epoxy resin curing agent, yields an epoxy resin cured product having excellent adhesion to inorganic substances, particularly aluminum, and an epoxy resin curing agent containing the reaction composition, an epoxy resin composition, and a cured product thereof.

### Solution to Problem

The present inventors have found that the above object can be achieved by a reaction composition containing a reaction product of xylylenediamine and a (meth)acrylamide derivative having only one (meth)acrylic group.

Specifically, the present invention relates to the following aspects.
[1] A reaction composition containing a reaction product of xylylenediamine and a (meth)acrylamide derivative having only one (meth)acrylic group.
[2] The reaction composition according to [1], wherein the reaction composition is obtained by reacting 0.8 to 1.8 mol of the (meth)acrylamide derivative per 1 mol of xylylenediamine.
[3] The reaction composition according to [1] or [2], wherein the (meth)acrylamide derivative is at least one selected from the group consisting of N,N-dimethyl(meth)acrylamide, N-hydroxyethyl(meth)acrylamide, and 4-(meth)acryloylmorpholine.
[4] The reaction composition according to any one of [1] to [3], wherein the reaction product contains at least one reaction product represented by any of the following formulas (1) to (3): 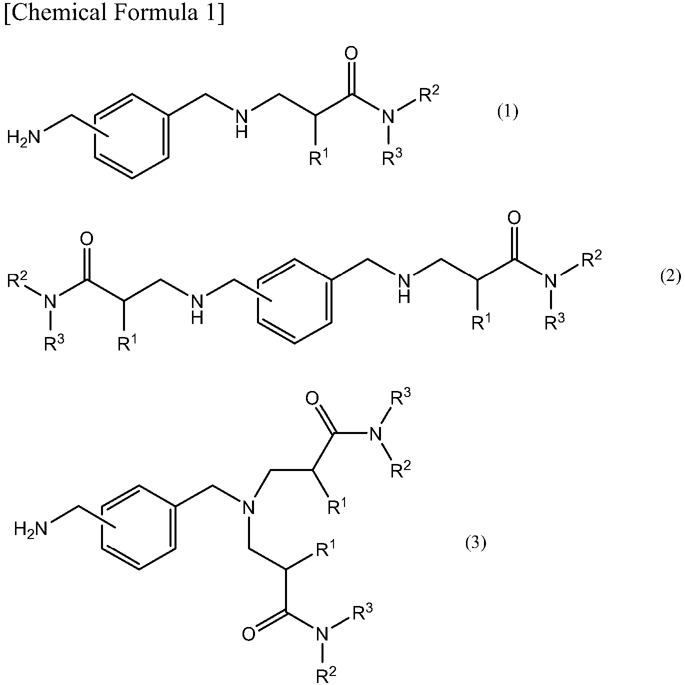 wherein R¹ is a hydrogen atom or a methyl group, R² and R³ are each independently a hydrogen atom, an alkyl group that has 1 to 8 carbon atoms and optionally has a hydroxy group, or a group represented by -(CH₂)ₙ-NR⁴R⁵ wherein n is a number of 1 to 10, and R⁴ and R⁵ are each independently a hydrogen atom or a methyl group, or R² and R³ together form a cyclic structure. R² and R³ are not both hydrogen atoms.
[5] An epoxy resin curing agent containing the reaction composition according to any one of [1] to [4].
[6] An epoxy resin composition containing an epoxy resin and the epoxy resin curing agent according to [5].
[7] A cured product obtained by curing the epoxy resin composition according to [6].

### Advantageous Effects of Invention

The present invention provides a reaction composition which, when used as an epoxy resin curing agent, yields an epoxy resin cured product having excellent adhesion to inorganic substances, particularly aluminum, and an epoxy resin curing agent containing the reaction composition, an epoxy resin composition, and a cured product thereof.

### Description of Embodiments

### [Reaction composition]

The reaction composition of the present invention contains a reaction product of xylylenediamine and a (meth)acrylamide derivative having only one (meth)acrylic group.

The "reaction composition containing a reaction product of xylylenediamine and a (meth)acrylamide derivative having only one (meth)acrylic group" herein means a product obtained by the reaction of xylylenediamine and the (meth)acrylamide derivative, which contains a reaction product (adduct) of the xylylenediamine and the (meth)acrylamide derivative. The reaction composition may contain an unreacted raw material and the like.

The (meth)acrylic group herein means an acrylic group or a methacrylic group.

The (meth)acrylamide derivative herein means an acrylamide derivative or a methacrylamide derivative. Acrylamide and methacrylamide are excluded from the (meth)acrylamide derivatives.

When the reaction composition of the present invention is used as an epoxy resin curing agent, an epoxy resin cured product having excellent adhesion to inorganic substances, particularly aluminum, can be obtained.

The reason why the above effect is obtained by the reaction composition of the present invention is not clear, but is considered as follows.

The reaction composition of the present invention is a type of modified product of xylylenediamine. By modifying xylylenediamine with a (meth)acrylamide derivative, a modified product (reaction product) having an increased molecular weight and containing an amide bond introduced therein is obtained. It is considered that by using this modified product as an epoxy resin curing agent, an epoxy resin cured product having excellent adhesion to inorganic substances, particularly aluminum, is obtained.

Xylylenediamine is at least one selected from the group consisting of o-xylylenediamine, m-xylylenediamine, and p-xylylenediamine. From the viewpoint of improving the adhesion to inorganic substances of the cured product of the epoxy resin composition obtained when the reaction composition is used as an epoxy resin curing agent, xylylenediamine is preferably at least one selected from the group consisting of m-xylylenediamine and p-xylylenediamine, more preferably m-xylylenediamine or a mixture of m-xylylenediamine and p-xylylenediamine, and further preferably m-xylylenediamine.

Examples of the (meth)acrylamide derivative having only one (meth)acrylic group (hereinafter sometimes simply referred to as "(meth)acrylamide derivative") include compounds represented by the following formula (I). 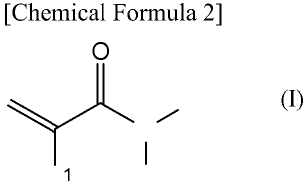 wherein R¹ is a hydrogen atom or a methyl group, R² and R³ are each independently a hydrogen atom, an alkyl group that has 1 to 8 carbon atoms and optionally has a hydroxy group, or a group represented by -(CH₂)ₙ-NR⁴R⁵ wherein n is a number of 1 to 10, and R⁴ and R⁵ are each independently a hydrogen atom or a methyl group, or R² and R³ together form a cyclic structure. R² and R³ are not both hydrogen atoms.

In formula (I), R¹ is preferably a hydrogen atom from the viewpoint of improving the adhesion to inorganic substances of the cured product of the epoxy resin composition obtained when the reaction composition is used as an epoxy resin curing agent.

Examples of alkyl groups that have 1 to 8 carbon atoms and optionally have a hydroxy group of R² and R³ in the formula (I) include a methyl group, an ethyl group, an n-propyl group, an isopropyl group, an n-butyl group, a sec-butyl group, a pentyl group, a hexyl group, a heptyl group, an octyl group, a hydroxymethyl group, a hydroxyethyl group, a hydroxypropyl group, a hydroxybutyl group, a hydroxypentyl group, a hydroxyhexyl group, a hydroxyheptyl group, and a hydroxyoctyl group. Among these, from the viewpoint of improving the adhesion to inorganic substances of the cured product of the epoxy resin composition obtained when the reaction composition is used as an epoxy resin curing agent, an alkyl group that has 1 to 6 carbon atoms and optionally has a hydroxy group is preferred, an alkyl group that has 1 to 4 carbon atoms and optionally has a hydroxy group is more preferred, and a methyl group, an ethyl group, or a hydroxyethyl group are further preferred.

In -(CH₂)ₙ-NR⁴R⁵ (n is a number of 1 to 10, and R⁴ and R⁵ are preferably both a methyl group) in R² and R³ of the formula (I), n is preferably from 2 to 6, more preferably from 2 to 4. R⁴ and R⁵ are preferably both a methyl group.

When R² and R³ of the formula (I) are groups that together form a cyclic structure, the cyclic structure is preferably a five-membered ring or a six-membered ring, and more preferably a six-membered ring, from the viewpoint of stability. The cyclic structure may contain a hetero atom such as a nitrogen atom or an oxygen atom as a ring member atom, and preferably contains an oxygen atom.

From the viewpoint of improving the adhesion to inorganic substances of the cured product of the epoxy resin composition obtained when the reaction composition is used as an epoxy resin curing agent, the cyclic structure is more preferably a morpholine ring.

Examples of the (meth)acrylamide derivative include N,N-dimethyl(meth)acrylamide, N,N-diethyl(meth)acrylamide, N-isopropyl(meth)acrylamide, N-hydroxyethyl(meth)acrylamide, N,N-dimethylaminopropyl(meth)acrylamide, and 4-(meth)acryloylmorpholine.

Among these, from the viewpoint of improving the adhesion to inorganic substances of the cured product of the epoxy resin composition obtained when the reaction composition is used as an epoxy resin curing agent, the (meth)acrylamide derivative is preferably at least one selected from the group consisting of N,N-dimethyl(meth)acrylamide, N-hydroxyethyl(meth)acrylamide, and 4-(meth)acryloylmorpholine, more preferably at least one selected from the group consisting of N,N-dimethylacrylamide, N-hydroxyethyl acrylamide, and 4-acryloylmorpholine, and further preferably at least one selected from the group consisting of N-hydroxyethyl acrylamide and 4-acryloylmorpholine.

The reaction product of xylylenediamine and a (meth)acrylamide derivative having only one (meth)acrylic group (hereinafter sometimes simply referred to as "reaction product") preferably contains at least one reaction product represented by any of the following formulas (1) to (3), and more preferably contains a reaction product represented by the following formula (1). The reaction products represented by any of the following formulas (1) to (3) are preferred because each of them has a hydrogen atom (active hydrogen) bonded to a nitrogen atom, and functions effectively as an epoxy resin curing agent. 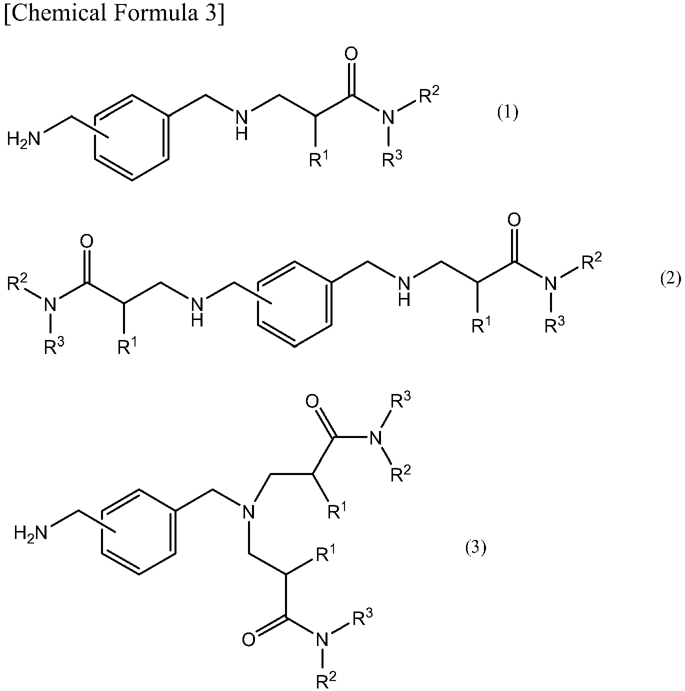 wherein R¹ is a hydrogen atom or a methyl group, R² and R³ are each independently an alkyl group that has 1 to 8 carbon atoms and optionally having a hydroxy group, or a group represented by -(CH₂)ₙ-NR⁴R⁵ wherein n is a number of 1 to 10, and R⁴ and R⁵ are each independently a hydrogen atom or a methyl group, or R² and R³ together form a cyclic structure. R² and R³ are not both hydrogen atoms.

R¹ to R³ and their preferred embodiments in the formulas (1) to (3) are the same as described above.

The reaction product represented by the formula (1) (hereinafter also referred to as "monoadduct") is the product of reacting xylylenediamine and the (meth)acrylamide derivative at a molar ratio of 1:1. The reaction product represented by each of the formulas (2) and (3) is the product of reacting xylylenediamine and the (meth)acrylamide derivative at a molar ratio of 1:2 (hereinafter also referred to as "diadducts").

The reaction composition may further contain a reaction product of reacting xylylenediamine and the (meth)acrylamide derivative at a molar ratio of 1:3 (triadduct), and a reaction product of reacting xylylenediamine and the (meth)acrylamide derivative at a molar ratio of 1:4 (tetraadduct).

However, from the viewpoint of allowing the reaction composition to function effectively as an epoxy resin curing agent, it is preferable that the reaction composition contains at least the reaction product represented by the formula (1).

The content of the reaction product of xylylenediamine and the (meth)acrylamide derivative having only one (meth)acrylic group in the reaction composition is preferably 50% by mass or more, more preferably 60% by mass or more, and further preferably 70% by mass or more, and 100% by mass or less, from the viewpoint of improving the adhesion to inorganic substances of the cured product of the epoxy resin composition obtained when the reaction composition is used as an epoxy resin curing agent.

The reaction composition may contain a by-product other than the reaction product, and an unreacted raw material such as xylylenediamine. However, from the viewpoint of improving the adhesion to inorganic substances of the cured product of the epoxy resin composition obtained when the reaction composition is used as an epoxy resin curing agent, it is preferable that the content of xylylenediamine in the reaction composition is small, and preferably 30% by mass or less.

The content of the unreacted raw material such as xylylenediamine in the reaction composition can be measured by gas chromatography (GC) analysis.

### <Viscosity>

The viscosity of the reaction composition at 25°C is preferably 50 Pa·s or less, more preferably 30 Pa·s or less, and further preferably 20 Pa·s or less, from the viewpoint of improving handleability. The lower limit of the viscosity is not particularly limited, but is preferably 10 mPa·s or more, more preferably 30 mPa·s or more.

The viscosity of the reaction composition at 25°C can be measured using an E-type viscometer, specifically by the method described in Examples.

### <Active Hydrogen Equivalent Weight (AHEW)>

The active hydrogen equivalent weight (AHEW) of the reaction composition is preferably 75 or more from the viewpoint of fast curing when the reaction composition is used as an epoxy resin curing agent and improving the adhesion to inorganic substances of the cured product of the epoxy resin composition obtained. The active hydrogen equivalent weight (AHEW) of the reaction composition is preferably 150 or less, more preferably 120 or less, and further preferably 100 or less from the viewpoint of improving curability. The active hydrogen equivalent weight (hereinafter also referred to as "AHEW") in the present description means the mass per mole of active hydrogen derived from the amino group in the reaction composition. The AHEW of the reaction composition can be calculated from the amine value of the reaction composition, and can be determined specifically by the method described in Examples.

### <Method for producing reaction composition>

The reaction composition of the present invention can be produced, for example, by subjecting xylylenediamine and the (meth)acrylamide derivative to an addition reaction under heating conditions.

From the viewpoint of increasing the content ratio of the reaction product of reacting xylylenediamine and the (meth)acrylamide derivative at a molar ratio of 1:1 (monoadduct) in the reaction composition, the reaction composition is preferably obtained by reacting 0.8 to 1.8 mol, more preferably 0.9 to 1.8 mol, further preferably 1.0 to 1.5 mol, and still more preferably 1.0 to 1.3 mol of the (meth)acrylamide derivative per 1 mol of xylylenediamine. Here, the molar amount refers to the molar amounts of xylylenediamine and the (meth)acrylamide derivative charged at the time of the reaction.

The reaction of xylylenediamine and the (meth)acrylamide derivative is preferably carried out under heating and stirring.

In the addition reaction, for example, xylylenediamine is charged into a reaction vessel, the (meth)acrylamide derivative is added dropwise thereto while stirring, and, after completion of the dropping, the mixture is heated preferably to 50 to 150°C, more preferably 70 to 130°C, and is reacted for 0.5 to 12 hours, thereby producing the reaction composition.

The addition reaction may also be carried out in a reaction solvent, but from the viewpoint of the improvement in reaction yield, it is preferably carried out without a solvent. The addition reaction is preferably performed under an inert gas atmosphere such as nitrogen gas.

After completion of the reaction, the resulting reaction liquid may be used directly as the reaction composition of the present invention, or may be distilled and purified to remove unreacted raw materials.

### [Epoxy resin curing agent]

An epoxy resin curing agent of the present invention contains the reaction composition of the present invention. When the epoxy resin curing agent is used for an epoxy resin composition, an epoxy resin cured product having high adhesion to inorganic substances can be formed.

The content of the reaction composition in the epoxy resin curing agent is preferably 50% by mass or more, more preferably 60% by mass or more, further preferably 70% by mass or more, even more preferably 80% by mass or more, and yet more preferably 90% by mass or more, and is 100% by mass or less from the viewpoint of improving the adhesion to inorganic substances of the cured product of the epoxy resin composition obtained.

The epoxy resin curing agent of the present invention may contain another curing agent component in addition to the above-described reaction composition. The curing agent component herein means a component that is contained in the epoxy resin curing agent and has two or more active hydrogen atoms that may react with epoxy groups in an epoxy resin.

Examples of the another curing agent component include an amine-based curing agent, a phenol-based curing agent, and an acid anhydride-based curing agent. From the viewpoint of fast curing, an amine-based curing agent is preferred.

Examples of the amine-based curing agent include a polyamine compound other than the reaction composition and a modified product thereof. The polyamine compound is not limited as long as it is a compound having at least two amino groups in its molecule.

Examples of the polyamine compound or the modified product thereof include: chain-like aliphatic polyamine compounds such as ethylenediamine, diethylenetriamine, triethylenetetramine, tetraethylenepentamine, pentaethylenehexamine, hexamethylenediamine, 2-methylpentamethylenediamine, and trimethylhexamethylenediamine; polyamine compounds having an alicyclic structure, such as 1,2-bis(aminomethyl)cyclohexane, 1,3-bis(aminomethyl)cyclohexane, 1,4-bis(aminomethyl)cyclohexane, menthenediamine, isophoronediamine, norbornanediamine, tricyclodecanediamine, adamantanediamine, diaminocyclohexane, 1,4-diamino-2-methylcyclohexane, 1,4-diamino-3,6-diethylcyclohexane, diaminodiethylmethylcyclohexane, 3,3'-dimethyl-4,4'-diaminodicyclohexylmethane, and 4,4'-diaminodicyclohexylmethane; polyamine compounds having an aromatic ring, such as o-xylylenediamine, m-xylylenediamine, p-xylylenediamine, phenylenediamine, diaminodiphenylmethane, and diaminodiphenylsulfone; polyamine compounds having a heterocyclic structure, such as N-aminomethylpiperazine and N-aminoethylpiperazine; polyether polyamine compounds; a reaction product of the above-mentioned polyamine compound and an epoxy compound having at least one epoxy group, an unsaturated hydrocarbon compound, a carboxylic acid, or a derivative thereof; a Mannich reaction product obtained by reacting the above-mentioned polyamine compound, a phenolic compound, and an aldehyde compound; and ketoimines (ketimines) obtained by reacting the above-mentioned polyamine compound and a ketone compound. These may be used singly or in combination of two or more of them.

When the another curing agent component is used, the content of the another curing agent component in the epoxy resin curing agent is preferably 1 mass% or more, more preferably 5 mass% or more. The upper limit of the content is not limited as long as the effects of the present invention are not impaired, but is preferably 50 mass% or less, more preferably 40 mass% or less, even more preferably 30 mass% or less, still even more preferably 20 mass% or less, still even more preferably 10 mass% or less.

The epoxy resin curing agent of the present invention may further contain a publicly-known curing accelerator, a non-reactive diluent, and others.

Examples of the curing accelerator include phenolic compounds, organic acids, organic acid salts, tertiary amines, quaternary ammonium salts, imidazoles, organophosphorus-based compounds, quaternary phosphonium salts, diazabicycloalkenes, organometallic salt compounds, boron compounds, and metallic halides.

Examples of the non-reactive diluent include benzyl alcohol, furfuryl alcohol, tetrahydrofurfuryl alcohol, and aromatic hydrocarbon formaldehyde resins, and any one or two or more of them may be used.

### <Active Hydrogen Equivalent Weight (AHEW)>

The active hydrogen equivalent weight (AHEW) of the epoxy resin curing agent is preferably 50 or more, more preferably 60 or more, and further preferably 75 or more from the viewpoint of fast curing when the reaction composition is used as an epoxy resin curing agent and improving the adhesion to inorganic substances of the cured product of the epoxy resin composition obtained. The active hydrogen equivalent weight (AHEW) of the epoxy resin curing agent is preferably 300 or less, more preferably 200 or less, further preferably 150 or less, even more preferably 120 or less, and yet more preferably 100 or less from the viewpoint of improving curability.

The AHEW of the epoxy resin curing agent may be calculated by the same method as that used for the AHEW of the reaction composition.

### [Epoxy resin composition]

An epoxy resin composition of the present invention contains an epoxy resin and the above-described epoxy resin curing agent. The cured product of the epoxy resin composition exhibits excellent adhesion to inorganic substances, particularly aluminum.

### <Epoxy resin>

The epoxy resin as a main agent of the epoxy resin composition may be any one of a saturated or unsaturated aliphatic compound or alicyclic compound, an aromatic compound, and a heterocyclic compound. An epoxy resin having an aromatic ring or an alicyclic structure in the molecule is preferred from the viewpoint of improving curing rate and improving the adhesion of the resulting cured product to inorganic substances.

Specific examples of such an epoxy resin include at least one resin selected from the group consisting of an epoxy resin having a glycidylamino group derived from m-xylylenediamine, an epoxy resin having a glycidylamino group derived from p-xylylenediamine, an epoxy resin having a glycidylamino group derived from 1,3-bis(aminomethyl)cyclohexane, an epoxy resin having a glycidylamino group derived from 1,4-bis(aminomethyl)cyclohexane, an epoxy resin having a glycidylamino group derived from diaminodiphenylmethane, an epoxy resin having a glycidylamino group and/or a glycidyloxy group derived from p-aminophenol, an epoxy resin having a glycidyloxy group derived from bisphenol A, an epoxy resin having a glycidyloxy group derived from bisphenol F, an epoxy resin having a glycidyloxy group derived from hydrogenated bisphenol A, an epoxy resin having a glycidyloxy group derived from hydrogenated bisphenol F, an epoxy resin having a glycidyloxy group derived from phenol novolac, and an epoxy resin having a glycidyloxy group derived from resorcinol. The above-mentioned epoxy resins may be used in combination of two or more of them.

Among these, from the viewpoint of obtaining a curing rate and the viewpoint of improving the adhesion of the resulting cured product to inorganic substances, preferred is an epoxy resin containing, as its main component, at least one selected from the group consisting of an epoxy resin having a glycidylamino group derived from m-xylylenediamine, an epoxy resin having a glycidylamino group derived from p-xylylenediamine, an epoxy resin having a glycidyloxy group derived from bisphenol A, and an epoxy resin having a glycidyloxy group derived from bisphenol F, and from the viewpoint of improving a curing rate, the viewpoint of improving the adhesion of the resulting cured product to inorganic substances and the viewpoint of availability and economy, more preferred is an epoxy resin containing, as its main component, an epoxy resin having a glycidyloxy group derived from bisphenol A.

It should be noted that the term "main component" here means that another component may be contained without departing from the spirit of the present invention, and means that the content thereof is preferably 50 to 100 mass%, more preferably 70 to 100 mass%, even more preferably 90 to 100 mass% of the total mass.

From the viewpoint of improving handleability, the epoxy resin as a main agent may contain a reactive diluent other than the above-described epoxy resin. The reactive diluent may be a low-molecular-weight compound having at least one epoxy group, and examples thereof include: aromatic monoglycidyl ethers such as phenylglycidyl ether and cresylglycidyl ether; alkylmonoglycidyl ethers such as butylglycidyl ether, hexylglycidyl ether, octylglycidyl ether, decylglycidyl ether, laurylglycidyl ether, and tetradecylglycidyl ether; and diglycidyl ethers of aliphatic diols, such as 1,3-propanedioldiglycidyl ether, 1,4-butanedioldiglycidyl ether, neopentylglycoldiglycidyl ether, and 1,6-hexanedioldiglycidyl ether.

The above-mentioned reactive diluents may be used singly or in combination of two or more of them.

The content ratio between the epoxy resin and the epoxy resin curing agent in the epoxy resin composition of the present invention is set in such a manner that the ratio of the number of amino group-derived active hydrogen atoms in the epoxy resin curing agent to the number of epoxy groups in the epoxy resin (the number of amino group-derived active hydrogen atoms in the epoxy resin curing agent/the number of epoxy groups in the epoxy resin) is preferably 1/0.5 to 1/2, more preferably 1/0.75 to 1/1.5, even more preferably 1/0.8 to 1/1.2.

The epoxy resin content and the epoxy resin curing agent content in the epoxy resin composition are not limited as long as the ratio of the number of amino group-derived active hydrogen atoms in the epoxy resin curing agent to the number of epoxy groups in the epoxy resin is preferably within the above range. However, from the viewpoint of improving a curing rate and the viewpoint of improving the adhesion of the resulting cured product to inorganic substances, the epoxy resin content and the epoxy resin curing agent content in the epoxy resin composition are preferably within the following ranges, respectively.

The epoxy resin content in the epoxy resin composition is preferably 40 mass% or more, more preferably 50 mass% or more, even more preferably 60 mass% or more and is preferably 80 mass% or less, more preferably 75 mass% or less.

The epoxy resin curing agent content in the epoxy resin composition is preferably 20 mass% or more, more preferably 25 mass% or more and is preferably 60 mass% or less, more preferably 50 mass% or less, even more preferably 40 mass% or less.

Further, the epoxy resin curing agent content in the epoxy resin composition is preferably 20 to 60 parts by mass, more preferably 30 to 60 parts by mass, still even preferably 40 to 60 parts by mass per 100 parts by mass of the epoxy resin as a main agent.

From the viewpoint of improving the adhesion of the resulting cured product to inorganic substances, the total content of the epoxy resin and the epoxy resin curing agent in the epoxy resin composition is preferably 40 mass% or more, more preferably 50 mass% or more, even more preferably 60 mass% or more, still even more preferably 70 mass% or more, still even more preferably 80 mass% or more, still even more preferably 90 mass% or more and is 100 mass% or less.

The epoxy resin composition of the present invention may further contain a modifying component such as a filler or a plasticizer, a flow modifying component such as a thixotropic agent, and another component such as a pigment, a leveling agent, a tackifier, or elastomer fine particles depending on its intended use.

### <Method for producing epoxy resin composition>

A method for producing the epoxy resin composition of the present invention is not limited, and the epoxy resin composition of the present invention can be produced by mixing the epoxy resin, the epoxy resin curing agent, and another optional component by a publicly-known method using a publicly-known device. The order of mixing the components of the epoxy resin composition is not limited, either. The epoxy resin curing agent may be prepared first and then mixed with the epoxy resin, or the components of the epoxy resin curing agent, another component, and the epoxy resin may be mixed at the same time.

### [Cured product]

A cured product of the epoxy resin composition of the present invention (hereinafter simply referred to also as a "cured product of the present invention") is obtained by curing the above-described epoxy resin composition. The curing conditions of the epoxy resin composition are not limited and are appropriately selected depending on the intended use and form of a resulting cured product. For example, the curing temperature of the epoxy resin composition may be selected from 10 to 150°C, and the curing time may be selected from 0.5 minutes to 7 days.

The form of the cured product of the present invention is not limited, either and may appropriately be selected depending on the intended use of the cured product. From the viewpoint of forming an epoxy resin cured product having excellent adhesion to inorganic substances, particularly aluminum, the cured product of the epoxy resin composition is preferably film-like or plate-like.

### <Intended use>

The epoxy resin composition of the present invention can form an epoxy resin cured product having excellent adhesion to inorganic substances and therefore can be used in various applications such as paints or adhesives for concrete, cement mortar, metals, or glass; pressure-sensitive adhesives for labels, wallpapers, and flooring materials; fiber-treating agents for glass fibers, carbon fibers, or metal fibers; building materials such as sealing materials, cement admixtures, and waterproofing materials; and other wide-ranging applications.

### Examples

The present invention will be described below in detail with reference to examples and comparative examples, but the present invention is not limited to the following examples. It should be noted that amino compositions were analyzed and evaluated by the following methods.

### <Gas chromatographic (GC) analysis>

The amount of residual m-xylylenediamine in the reaction composition (and, in Comparative Example 3, the composition of the comparative reaction composition F) was analyzed by GC. The GC measurement conditions are shown below.
Device: "7890B GC" manufactured by Agilent Technologies, Inc.
Column: "CP-Sil 8 CB for Amines" manufactured by Agilent Technologies, Inc. (length 30 m, film thickness 0.25 µm, inner diameter 0.25 mm)
Column temperature: 40°C 10 min → heating up at 20°C/min → 250°C 10 min → heating up at 20°C/min → 300°C 10 min
Carrier gas: helium
Carrier gas flow rate: 2.2553 mL/min
Inlet pressure: 22.474 psi (constant pressure mode)
Detector: FID
Inlet temperature: 250°C
Detector temperature: 310°C

### <FD-MS Analysis>

The measurement conditions for field-desorption mass spectrometry (FD-MS) of the reaction composition are as follows.
Measuring instrument: AccuTOF GCV 4G (JMS-T100GCV), JEOL
Ionization mode: Field Desorption (Positive)
Range: m/z = 30 to 800
Emitter current: 0 mA to 40 mA, 51.2 mA/min
Resolution: 8,000 (at m/z = 501.9711 (C₉F₂₀N))
Counter electrode voltage: -10 kV
Detector voltage: 2,000 V
Sampling interval: 0.25 ns
Recording interval: 0.5 s
Drift compensation: m/z = 501.9711 (C₉F₂₀N)

### <¹H-NMR Analysis>

The measurement conditions for ¹H-NMR of the reaction composition are as follows.
Nuclear magnetic resonance spectrometer: AVANCEIII-500 manufactured by Bruker BioSpin GmbH.
Probe: 5 mmϕ double resonance, multinuclear probe (BBFO Plus Smart Probe)
Deuterated solvent: deuterated chloroform
Nuclide to be measured: 1H
Measurement temperature: room temperature

### <Viscosity>

The viscosity of a reaction composition (curing agent) at 25°C was measured using a type-E viscometer "TVE-35H viscometer cone plate type" manufactured by Toki Sangyo Co., Ltd. In Table 1, the viscosity of Amine D (MXDA) was measured at 20°C.

### <Active hydrogen equivalent weight (AHEW)>

The AHEW of a reaction composition (curing agent) was calculated from the results of determination of a total amine value and a secondary/tertiary amine value with the use of an automatic potentiometric titrator "AT-710S" manufactured by Kyoto Electronics Manufacturing Co., Ltd. The total amine value was measured using a 0.1 mol/L perchloric/acetic acid solution (manufactured by KANTO CHEMICAL CO., INC.), and the secondary/tertiary amine value was measured using 0.1 mol/L hydrochloric acid (2-propanol). In Table 1, regarding some reaction composition (curing agent), theoretical AHEW values are shown for certain curing agents.

### <Adhesion strength to aluminum substrate>

Test specimens having the shape specified in JIS K6851:1994 were prepared as follows.

Each epoxy resin composition of Examples was applied to one surface of an aluminum base material 25 mm × 100 mm × 1.6 mm thick ("A1050P" manufactured by Standard Test Piece) using a wooden spatula (coating film thickness: 200 µm). This was heat-cured for 1 hour in a hot-air dryer set at 120°C to prepare a test specimen having a cured product layer of the epoxy resin composition formed on one surface of the aluminum base material.

Next, using the test specimen, a tensile shear test was performed under the conditions of 23°C and 50% RH in accordance with JIS K6851:1994 using an autograph tester manufactured by Shimadzu Corporation at a stroke speed of 1 mm/min (n = 3). Values obtained by dividing the fracture load (maximum load, unit: N) when the cured product layer separated from the base material by the shear area (unit: mm²) of the test piece are shown in Table 1 as the adhesion strength.

The components used in the following Examples and Comparative Examples are as follows.
· MXDA: m-xylylenediamine, manufactured by Mitsubishi Gas Chemical Company, Inc.
· HEAA: N-hydroxyethyl acrylamide, manufactured by FUJIFILM Wako Pure Chemical Corporation.
· ACMO: 4-acryloylmorpholine, manufactured by Tokyo Chemical Industry Co., Ltd.
· DMAA: N,N-dimethylacrylamide, manufactured by Tokyo Chemical Industry Co., Ltd.
· G-328: a reaction product of m-xylylenediamine and epichlorohydrin, manufactured by Mitsubishi Gas Chemical Company, Inc., "Gaskamine 328"
· BAL: benzaldehyde, manufactured by Tokyo Chemical Industry Co., Ltd.

### Example 1 (Production and evaluation of reaction composition A containing reaction product of MXDA and HEAA)

### (Production of reaction composition A)

A separable flask having an internal capacity of 300 milliliters and equipped with a stirrer, a thermometer, a nitrogen inlet tube, a dropping funnel, and a condenser was charged with 40.8 g (0.3 mol) of m-xylylenediamine (MXDA), and 34.5 g (0.3 mol) of hydroxyethyl acrylamide (HEAA) was added dropwise thereto over 60 minutes with stirring under nitrogen stream. After completion of the dropping, the temperature was increased to 70°C and the reaction was carried out for 120 minutes. The temperature was further increased to 100°C and the mixture was stirred for 30 minutes to obtain a reaction composition A containing the reaction product of MXDA and HEAA.

The resulting reaction composition A was subjected to FD-MS and ¹H-NMR analyses under the aforementioned conditions.

As a result of the FD-MS analysis, peaks corresponding to the unreacted raw material, m-xylylenediamine, as well as to the compound represented by the following formula (1A) (monoadduct), the compound represented by the following formula (2A) (diadduct), the compound represented by the following formula (3A) (diadduct), and the compound represented by the following formula (4A) (triadduct) were detected. Specifically, the peaks corresponding to each compound were identified as follows.
· m/z = 137.10: m-xylylenediamine (Mw 136.2)
· m/z = 252.15: compound represented by the following formula (1A) (Mw 251.33)
· m/z = 367.20: compound represented by the following formula (2A) and compound represented by formula (3A) (Mw 366.46)
· m/z = 482.26: compound represented by the following formula (4A) (Mw 481.59)
· m/z = 503.29: ionized species of dimer of compound represented by the following formula (1A)

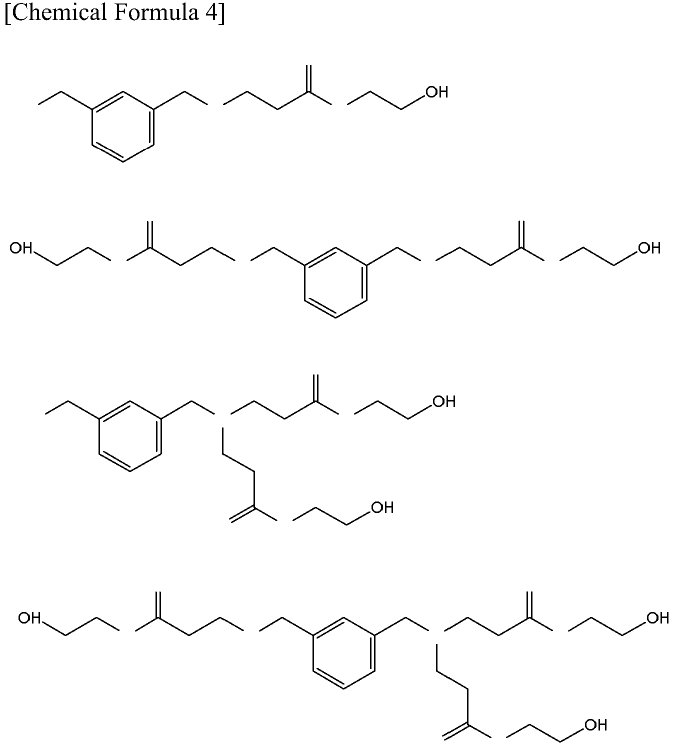

¹H-NMR chemical shifts: δ 7.33-7.10 ppm (m, -C₆H₄-), 3.92 ppm (s, -NH₂-CH₂-C₆H4-), 3.87 ppm(s, -NH₂-CH₂-C₆H₄-), 3.80 ppm (d, J = 9.0 Hz, -NH-CH₂-C₆H₄-), 3.68 (dt, J = 10 Hz, 5 Hz, -NH-CH₂-CH₂-), 3.43-3.34 ppm (m, -CH₂-CH₂-CO-), 2.94 ppm (t, J = 5.75 Hz, -NH-CH₂-CH₂-), 2.41 ppm (td, J = 5.75Hz, 3.0 Hz, -CH₂-CH₂-OH), 2.17 ppm(br, -CH₂-NH-CO-, -CH₂-NH-CH₂-)

In addition, 0.1 g of the resulting reaction composition A was dissolved in 1 g of methanol, and the reaction composition was analyzed by GC under the aforementioned conditions. The content of MXDA in the reaction composition A was 19.7% by mass.

### (Evaluation of reaction composition A)

Using the resulting reaction composition A, viscosity and AHEW were measured by the aforementioned method. Epoxy resin compositions were prepared using the reaction composition A as a curing agent, and evaluated by the following method.

### [Preparation and evaluation of epoxy resin composition]

A liquid epoxy resin having glycidyloxy groups derived from bisphenol A ("jER828" manufactured by Mitsubishi Chemical Corporation, epoxy equivalent weight 186 g/eq.) was used as a main agent epoxy resin, and the above-described reaction composition A was used as an epoxy resin curing agent.

The epoxy resin and the epoxy resin curing agent were blended according to a formulation shown in Table 1 and mixed with stirring at 23°C to prepare an epoxy resin composition. The ratio of the number of active hydrogen atoms in the epoxy resin curing agent to the number of epoxy groups in the main agent epoxy resin (the number of active hydrogen atoms in the epoxy resin curing agent/the number of epoxy groups in the main agent epoxy resin) was set to 1/1.

Using the resulting epoxy resin composition, adhesion strength to an aluminum base material was evaluated by the aforementioned method. The results are shown in Table 1.

### Example 2 (Production and evaluation of reaction composition B containing reaction product of MXDA and ACMO)

### (Production of reaction composition B)

The reaction composition B was produced in the same manner as in Example 1 except that 42.4 g (0.3 mol) of 4-acryloylmorpholine (ACMO) was used instead of HEAA.

The resulting reaction composition B was subjected to FD-MS and ¹H-NMR analyses under the aforementioned conditions.

As a result of the FD-MS analysis, peaks corresponding to the compound represented by the following formula (1B) (monoadduct), the compound represented by the following formula (2B) (diadduct), the compound represented by the following formula (3B) (diadduct), and the compound represented by the following formula (4B) (triadduct) were detected. Specifically, the peaks corresponding to each compound were identified as follows.
· m/z = 278.19: compound represented by the following formula (1B) (Mw 277.37)
· m/z = 419.27: compound represented by the following formula (2B) and compound represented by the following formula (3B) (Mw 418.26)
· m/z = 555.37: ionized species of dimer of compound represented by the following formula (1B)
· m/z = 560.34: compound represented by the following formula (4B) (Mw 559.34)
· m/z = 696.45: ionized species of heterodimer of monoadduct and diadduct

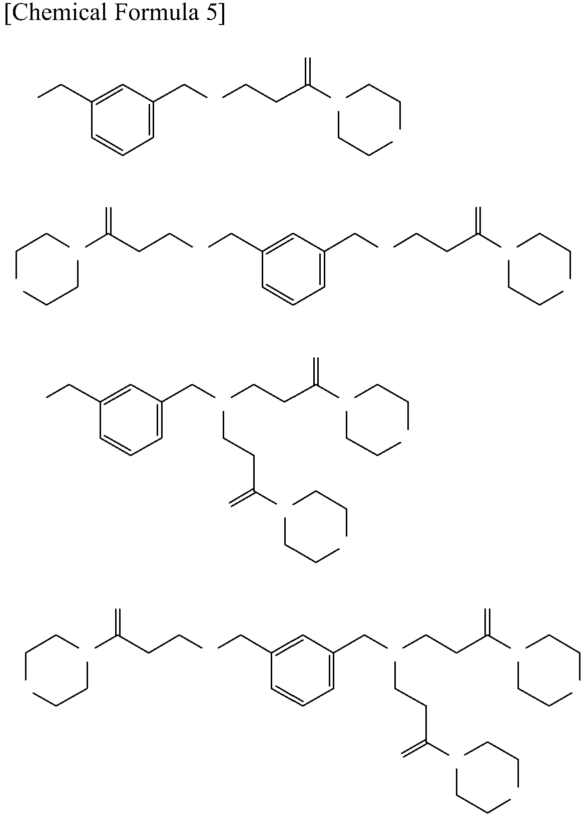

¹H-NMR chemical shifts: δ 7.34-7.07 ppm (m, -C₆H₄-), 3.87 ppm (d, J = 8.5 Hz, -NH₂-CH₂-C₆H₄-), 3.80 ppm (d, J = 7.5 Hz, -NH-CH₂-C₆H₄-), 3.67-3.63 ppm (m, -CH₂-CH₂-O-), 3.63-3.56 ppm (m, -N-CH₂-O-), 3.46-3.42 ppm (m, -N-CH₂-O-), 2.91 ppm (dt, J = 6.25 Hz, 3.0 Hz, -NH-CH₂-CH₂-), 2.53 ppm (dt, J = 6.25 Hz, 2.0 Hz, -CH₂-CH₂-CO-), 1.78 ppm (br, - CH₂-NH-CH₂-).

In addition, 0.1 g of the resulting reaction composition B was dissolved in 1 g of methanol, and the reaction composition was analyzed by GC under the aforementioned conditions. The content of MXDA in the reaction composition B was 23.1% by mass.

### (Evaluation of reaction composition B and preparation and evaluation of epoxy resin composition)

The reaction composition B was evaluated and epoxy resin compositions having the composition shown in Table 1 were prepared and evaluated in the same manner as in Example 1 except that the reaction composition B was used instead of the reaction composition A. The results are shown in Table 1.

### Example 3 (Production and evaluation of reaction composition C containing reaction product of MXDA and DMAA)

### (Production of reaction composition C)

The reaction composition C was produced in the same manner as in Example 1 except that the charge amount of m-xylylenediamine was changed to 33.0 g (0.24 mol) and 24.0 g (0.24 mol) of N,N-dimethylacrylamide (DMAA) was used instead of HEAA.

The resulting reaction composition C was subjected to FD-MS and ¹H-NMR analyses under the aforementioned conditions.

As a result of the FD-MS analysis, peaks corresponding to the compound represented by the following formula (1C) (monoadduct), the compound represented by the following formula (2C) (diadduct), and the compound represented by the following formula (3C) (diadduct) were detected. Specifically, the peaks corresponding to each compound were identified as follows.
· m/z = 236.14: compound represented by the following formula (1C) (Mw 235.33)
· m/z = 335.19: compound represented by the following formula (2C) and compound represented by the following formula (3C) (Mw 334.24)

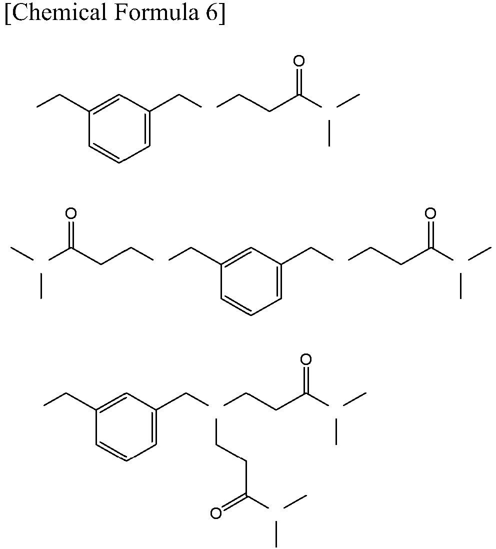

¹H-NMR chemical shifts: δ 7.35-7.14 ppm (m, -C₆H₄-), 3.86 ppm (d, J = 11 Hz, -NH₂-CH₂-C₆H₄-), 3.80 ppm (d, J = 7.5 Hz, -NH-CH₂-C₆H₄-), 3.00-2.96 ppm (m, -NH-CH₂-CH₂-), 2.96-2.92 ppm (m, -CH₂-CH₂-CO-), 2.92-2.88 ppm (m, -N-CH₃), 2.57-2.50 ppm (m, -N-CH₃), 1.67 ppm (br, -CH₂-NH-CH₂-).

In addition, 0.1 g of the resulting reaction composition C was dissolved in 1 g of methanol, and the reaction composition was analyzed by GC under the aforementioned conditions. The content of MXDA in the reaction composition C was 20.2% by mass.

### (Evaluation of reaction composition C and preparation and evaluation of epoxy resin composition)

The reaction composition C was evaluated and epoxy resin compositions having the composition shown in Table 1 were prepared and evaluated in the same manner as in Example 1 except that the reaction composition C was used instead of the reaction composition A. The results are shown in Table 1.

### Comparative Example 1

An epoxy resin composition having the composition shown in Table 1 was prepared and evaluated in the same manner as in Example 1 except that Amine D (MXDA) was used instead of the reaction composition A. The results are shown in Table 1. The viscosity and AHEW of MXDA are shown in Table 1.

### Comparative Example 2

An epoxy resin composition having the composition shown in Table 1 was prepared and evaluated in the same manner as in Example 1 except that Amine E (G-328: a reaction product of m-xylylenediamine and epichlorohydrin, "Gaskamine 328" manufactured by Mitsubishi Gas Chemical Company, Inc.) was used instead of the reaction composition A. The results are shown in Table 1. The viscosity and AHEW of G-328 are shown in Table 1.

### Comparative Example 3 (Production and evaluation of comparative reaction composition F containing hydrogenated product of reaction product of MXDA and BAL)

### (Production of comparative reaction composition F)

### [Step (1)]

A separable flask having an internal capacity of 300 milliliters and equipped with a stirrer, a thermometer, a nitrogen inlet tube, a dropping funnel, and a condenser was charged with 40.0 g (0.3 mol) of m-xylylenediamine (manufactured by Mitsubishi Gas Chemical Company, Inc.; MXDA). 31.9 g (0.3 mol) of benzaldehyde (manufactured by Tokyo Chemical Industry Co., Ltd.) was added dropwise over 15 to 20 minutes with stirring under nitrogen stream. After completion of the dropping, the temperature was increased to 80°C and the reaction was carried out for 30 minutes. ¹H-NMR of the reaction product was measured, and the disappearance of the aldehyde-derived ¹H peak of benzaldehyde was confirmed, thereby completing the reaction. Thereafter, toluene was added to the reaction product, and moisture in the system was removed by azeotropic dehydration to obtain an imine, which is the reaction product of m-xylylenediamine and benzaldehyde at a molar ratio of 1/1.

### [Step (2)]

An autoclave (capacity: 230 mL; material: SUS316L) equipped with a stirrer and a heater was charged with 30.3 g of the imine obtained in Step (1), 30 g of toluene, and 0.3 g of 5% Pd/C catalyst (manufactured by N. E. Chemcat Corporation; PE type) as a catalyst. The vapor phase was replaced with hydrogen. Then, temperature rise was started while stirring was performed to achieve a liquid temperature of 80°C in 1 hour, and then the pressure was increased to 1 MPaG with hydrogen. Then, a reaction was continued for 1.5 hours under the condition of a liquid temperature of 80°C while hydrogen was supplied as needed in such a manner that the pressure was maintained at 1 MPaG. The resulting reaction liquid was filtered to remove the hydrogenation catalyst, and then concentrated under reduced pressure to obtain 28.8 g of an amino composition f, which is a reduced product of the imine obtained in Step (1). Residual m-xylylenediamine was removed from the resulting amino composition f by distillation under reduced pressure to obtain 24.9 g of comparative reaction composition F.

In the comparative reaction composition F, the GC area ratios of the compound represented by the following formula (1F) (monoadduct) and the compound represented by the following formula (2F) (diadduct) were 74.7% and 25.3%, respectively.

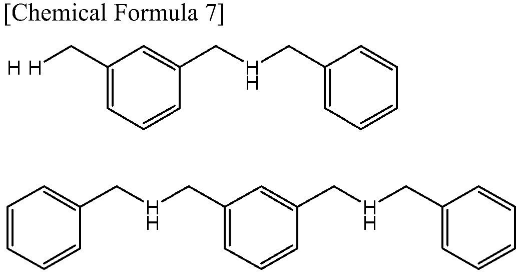

The structure of the compound represented by the formula (1F) (monoadduct) and the compound represented by the formula (2F) (diadduct) was analyzed by ¹H-NMR of the comparative reaction composition F.

¹H-NMR chemical shifts: δ 7.1-7.9 ppm (m, -C₆H₄- and -C₆H₅), δ 3.8-3.85 ppm (s, - CH₂-C₆H₄-CH₂- and -CH₂-C₆H₅), δ 1.9 ppm (s, -NH- and -NH₂).

### (Evaluation of comparative reaction composition F and preparation and evaluation of epoxy resin composition)

The comparative reaction composition F was evaluated and epoxy resin compositions having the composition shown in Table 1 were prepared and evaluated in the same manner as in Example 1 except that the comparative reaction composition F was used instead of the reaction composition A. The results are shown in Table 1.

### [Table 1]

**Table 1**

| (Parts by mass) | | | Example 1 | Example 2 | Example 3 | Comparative Example 1 | Comparative Example 2 | Comparative Example 3 |
|---|---|---|---|---|---|---|---|---|
| Epoxy resin composition | Curing agent | Reaction composition A (MXDA/HEAA) | 31 | | | | | |
| | | Reaction composition B (MXDA/ACMO) | | 33 | | | | |
| | | Reaction composition C (MXDA/DMAA) | | | 29 | | | |
| | | Amine D (MXDA) | | | | 15 | | |
| | | Amine E (G-328) | | | | | 23 | |
| | | Comparative reaction composition F (hydrogenated product of MXDA/BAL reaction product) | | | | | | 32 |
| | Main agent | jER828 | 69 | 67 | 71 | 85 | 77 | 68 |
| Results of evaluation | Viscosity of curing agent (25°C) (mPa · s) | | 19000 | 1500 | 73.4 | 6.8^{*2} | 8630 | 59.7 |
| | AHEW of curing agent (g/eq.) | | 83.7^{*1} | 92 | 77 | 34 | 55 | 89 |
| | Adhesion strength to aluminum base material (MPa) | | 5.60 | 3.50 | 2.13 | 1.68 | 0.98 | 0.83 |

| | | | | | | | | |
|---|---|---|---|---|---|---|---|---|
| *1: Theoretical value *2: Measured value at 20°C | | | | | | | | |

Table 1 shows that the cured product of the epoxy resin composition using the reaction composition of the present invention as an epoxy resin curing agent exhibits excellent adhesion to aluminum base materials.

### Industrial Applicability

The present invention provides a reaction composition which, when used as an epoxy resin curing agent, yields an epoxy resin cured product having excellent adhesion to inorganic substances, particularly aluminum, and an epoxy resin curing agent containing the reaction composition, an epoxy resin composition, and a cured product thereof.

## Claims

1. A reaction composition comprising a reaction product of xylylenediamine and a (meth)acrylamide derivative having only one (meth)acrylic group.

2. The reaction composition according to claim 1, wherein the reaction composition is obtained by reacting 0.8 to 1.8 mol of the (meth)acrylamide derivative per 1 mol of xylylenediamine.

3. The reaction composition according to claim 1 or 2, wherein the (meth)acrylamide derivative is at least one selected from the group consisting of N,N-dimethyl(meth)acrylamide, N-hydroxyethyl(meth)acrylamide, and 4-(meth)acryloylmorpholine.

4. The reaction composition according to any one of claims 1 to 3, wherein the reaction product comprises at least one reaction product represented by any of the following formulas (1) to (3): 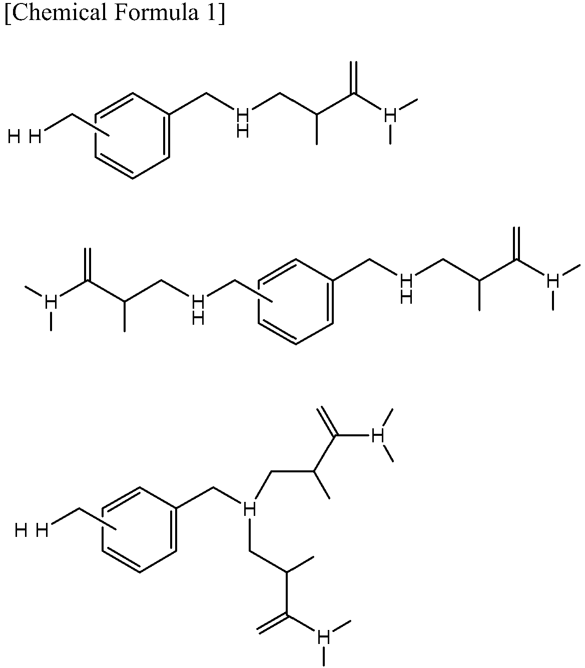 wherein R¹ is a hydrogen atom or a methyl group, R² and R³ are each independently a hydrogen atom, an alkyl group that has 1 to 8 carbon atoms and optionally has a hydroxy group, or a group represented by -(CH₂)ₙ-NR⁴R⁵ wherein n is a number of 1 to 10, and R⁴ and R⁵ are each independently a hydrogen atom or a methyl group, or R² and R³ together form a cyclic structure. R² and R³ are not both hydrogen atoms.

5. An epoxy resin curing agent comprising the reaction composition according to any one of claims 1 to 4.

6. An epoxy resin composition comprising an epoxy resin and the epoxy resin curing agent according to claim 5.

7. A cured product obtained by curing the epoxy resin composition according to claim 6.
